(19) **Europäisches Patentamt**
**European Patent Office**
**Office européen des brevets**

(11) **EP 2 783 523 B1**

(12) **EUROPEAN PATENT SPECIFICATION**

(45) Date of publication and mention
of the grant of the patent:
**22.08.2018 Bulletin 2018/34**

(51) Int Cl.:
*H04R 3/00* *(2006.01)*      *H04R 25/00* *(2006.01)*
*H03G 7/00* *(2006.01)*      *A61N 1/36* *(2006.01)*

(21) Application number: **12723645.3**

(86) International application number:
**PCT/EP2012/059492**

(22) Date of filing: **22.05.2012**

(87) International publication number:
**WO 2013/075848 (30.05.2013 Gazette 2013/22)**

(54) **SYSTEM AND METHOD FOR SIGNAL LEVEL DETECTION**

SYSTEM UND VERFAHREN ZUR SIGNALPEGELERKENNUNG

SYSTÈME ET PROCÉDÉ POUR LA DÉTECTION DE NIVEAU DE SIGNAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.11.2011 PCT/EP2011/070606**

(43) Date of publication of application:
**01.10.2014 Bulletin 2014/40**

(73) Proprietor: **Jacoti BVBA
8560 Wevelgem (BE)**

(72) Inventors:
• **NEUMANN, Joachim
E-08018 Barcelona (ES)**

• **MÉNDEZ RODRÍGUEZ, Nun
E-08860 Castelldefels (ES)**
• **KINSBERGEN, Jacques
B-2530 Boechout (BE)**
• **WACK, Nicolas
E-08025 Barcelona (ES)**

(74) Representative: **DenK iP
Hundelgemsesteenweg 1116
9820 Merelbeke (BE)**

(56) References cited:
**EP-A1- 1 491 068      EP-A2- 2 262 280**

**Description**

**Field of the invention**

The present invention is related to the field of signal level detection of an input audio signal of unknown level.

**Background of the invention**

Level detectors are found in various types of audio devices. They are the central piece in an Automatic Gain Control (AGC), which is sometimes also called dynamic range compressor or simply compressor.

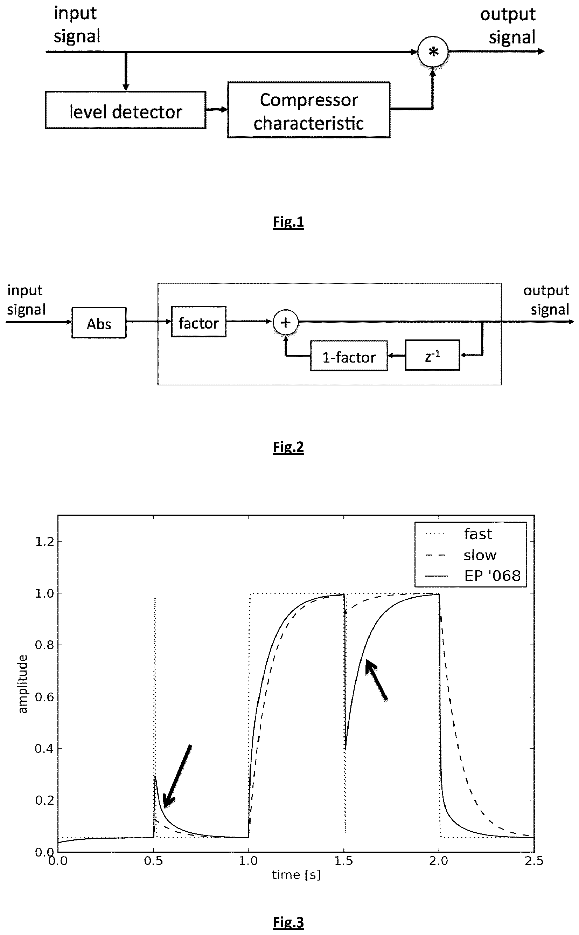

A simple feed-forward compressor is schematically shown in the block diagram of Fig.1. The compressor comprises a level detector, a compressor characteristic unit and a multiplier. The compressor output signal results from a multiplication of the input signal with a time-variant factor signal, which depends both on the input signal level and on the compressor characteristic.

The level detector produces a time-variant signal that estimates the input sound signal level. This level estimate can e.g. be based on the low-pass filtered rectified input sound signal or on the low-pass filtered squared signal to estimate the root-mean square value of the signal. This simple level detector is illustrated in Fig. 2. For a given sample rate sr and a time constant $\tau$ of the level detector, the factor applied in the scheme of Fig. 2 can be calculated as :

$$\text{factor} \ = \ e^{\frac{-1}{\tau \cdot \text{sr}}}$$

If the level detector is used to detect the level of audio signals, its output is typically converted to a logarithmic dB scale, for example to represent the level of the input signal in dB SPL (sound pressure level).

A level detector should on the one hand quickly follow the abrupt changes of the input signal level in order to allow for large gain changes as a reaction to changes in the input sound signal level. The level detector should on the other hand be as constant as possible to limit to a minimum the amount of distortion that comes with any gain change. This is the desired behaviour in situations in which the user needs to understand speech or is listening to music and the input signal does not exhibit very large level changes. The level detector together with the compressor characteristic unit thus determines the temporal properties and side effects of the dynamic range compressor shown in Fig.1.

One application field of level detectors is audio recording, mixing and mastering, e.g., for loudness maximization of audio for radio broadcasting. Another important application field of level detectors concerns hearing aids. Hearing aids and other hearing solutions need to adapt their amplification not only to the individual hearing loss of the patient, but they also need to be able to adapt the amount of amplification to the current sound environment. In hearing aids compressors are employed to provide more gain in soft listening situations than in loud listening situations. This is required for the majority of hearing impaired users who suffer from a sensorineural hearing loss and show the recruitment phenomenon.

Another application field of level detectors is the audio pre-processing in implantable auditory prostheses. For example, cochlear implants provide electric stimulation to the receptors and nerves in the human inner ear. In the signal processing chain of implantable auditory prostheses, the signal that is picked up by the microphone is processed in a similar fashion as in a hearing aid. In this first stage, level detectors are an important component to characterize the input signal and to reduce the dynamic range of the input signal. A second stage then transforms the optimized sound signal into an excitation pattern for the implanted prostheses.

If the AGC (compressor) provides more gain in a soft listening environment, protection is needed for sudden loud sounds. Therefore, the AGC should be capable of reducing the gain as fast as possible. However, fast gain changes introduce distortion and artefacts into the acoustic signal, which can reduce audio quality and speech intelligibility. Therefore, the AGC should preferably change the gain as little as necessary.

AGCs used in commercial products find a compromise between the two aforementioned requirements, typically by choosing a fast attack time (for increasing input sound levels) and a slow release time (for decreasing input sound levels). These attack and release time constants are part of the level detector and determine how fast the level detector follows an increasing input sound level and a decreasing input sound level, respectively.

The setting of the time constants in the level detector of a hearing instrument thus involves a compromise between the requirements of little distortion of speech on the one hand and on the other hand protection of the hearing impaired from sudden intense sounds. Traditionally, a fast attack time is used to provide protection and a long release time is used to reduce distortion effects. This compromise is not ideal because of (a) distortion of speech signals caused by the short attack time constants and (b) over-estimation of the level of dynamic signals such as speech due to long release time constants, resulting in too little gain. In addition, the user of a hearing instrument can in some cases hear that a background signal of constant level increases in intensity. This effect - which is sometimes called pumping - is caused by long release time constants resulting in a slow gain increase after a loud acoustical event.

Patent EP1491068 aims to tackle this by an input signal dependent optimal time constant. The described level detector is designed with the guiding principle of maximizing the use of long time constants while at the same time being able to detect abrupt input level changes. The dynamic behaviour of the level detector is based on a simple calculation scheme : the input signal is applied to an auxiliary level detector and in parallel to a so-called guided level detector. The auxiliary level detector has a fixed small time constant that allows it to react faster to changes in the input sound signal than the guided level detector. The guided level detector receives as input either the applied input sound signal or the auxiliary level detection output. The guided level detector output is also the output of the system as a whole. The time constant of the guided level detector and thus its dynamic behaviour is based on an analysis of the difference of the outputs from the auxiliary and the guided level detector itself.

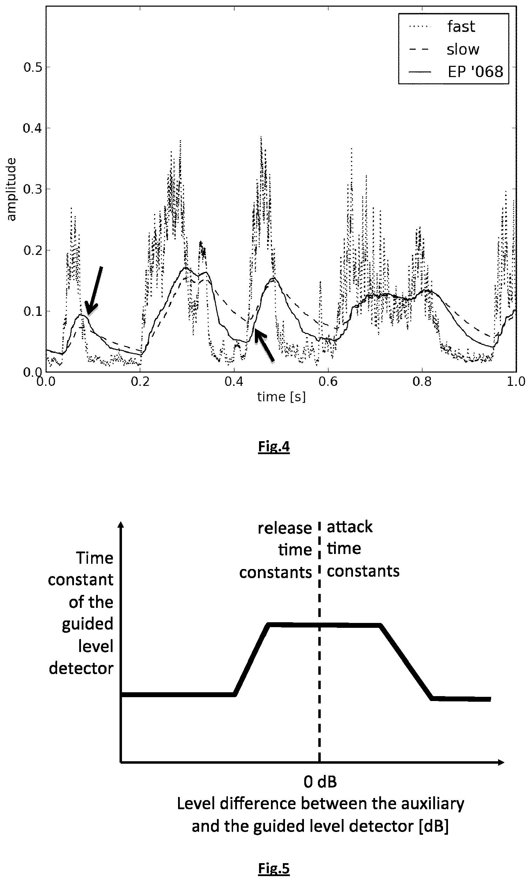

Through the proposed method, level detectors with various different characteristics can be realized based on how the relationship is defined between the output from the two level detectors and the time constants setting of the guided level detector. This is defined in the time constant function, as illustrated in Fig.5. This time constant function and the outputs of the two level detectors provide all the information necessary to set a time constant of the guided level detector, which together with a compressor characteristic unit and a multiplier yields a compressed signal.

The behaviour of the level detectors discussed in patent EP1491068 is illustrated with an artificial input signal (Fig.3). In the first part of the artificial signal of 1 second duration, the input level is low, but at 0.5 seconds, the low signal is interrupted by a sudden burst of 8 milliseconds duration, which is 25 dB larger. The second section of the artificial signal from 1 second to 2 seconds, the signal level is high, only interrupted by a sudden drop at 1.5 seconds of 8 milliseconds duration. Fig.3 shows the output signal of a fast level detector with a time constant of 2 milliseconds, plotted as dotted line ("fast"). This line follows the actual level of the input signal level (not shown) quite well. The output of the slow level detector that uses a time constant of 100 milliseconds is plotted as dashed line ("slow") as a function of time. In Fig.3, at around 0.5 seconds and at around 1.5 seconds, the dashed curve "slow" of the slow level detector is only slightly altered by the sudden rise or drop of the input signal.

The output of an implementation of the level detector described in EP1491068 is represented by the solid line ("EP '068"). In this implementation, the time constant function of EP1491068 ranges from 2 milliseconds to 100 milliseconds.

Also the behaviour of the level detectors to a speech signal is illustrated. A desired level detector behaviour is to produce an output that is close to the output of the fast level detector for signals with sudden level changes similar to those used in Fig.3. In contrast, for speech signals as in Fig.4 the desired level detector behaviour is to produce an output close to the slow level detector output. The solid lines ("EP '068") in Figs 3 and 4 illustrate indeed that the level detector described in EP1491068 is closer to the fast level detector in Fig.3 around 0.5 and 1.5 seconds, while it is closer to the slow level detector in Fig.4. at all times.

However, the solution of EP1491068 results in output values of the level detector as a whole that can lie outside the range defined by the fast and the slow level detector outputs. This can be observed around t = 0.6 sec and t = 1.6 sec in Fig.3 and around t = 0.09 sec and t = 0.5 sec in Fig.4 (indicated by arrows). As a consequence, a compressor based on the level detector as described in EP1491068 will overestimate the level of soft signals that follow sudden loud signals. For example, low-level speech signals that follow a door slam will receive too little amplification. Similarly, the level of loud signals that follow a sudden drop will be underestimated. As a consequence, loud signals that follow a short interruption will receive too much amplification. This phenomenon is known as overshoot. This undesired behaviour of the level detector described in EP1491068 occurs predominantly if the sudden level change is of short duration.

Hence, there is a need for a level detector wherein this drawback is avoided.

## Summary of the invention

It is an object of embodiments of the present invention to provide for a system and method for the detection of the level of an input signal to be used in a dynamic range compressor, whereby the level detector output remains within a range defined by the fast and slow level detector.

The above objective is accomplished by the solution according to the present invention.

In a first aspect the invention relates to a level detection system for estimating the level of an audio input signal to be used in a dynamic range compressor. The system comprises

- a first auxiliary level detector arranged for receiving an audio input signal and for outputting a first estimate of the level of the audio input signal,
- a second auxiliary level detector arranged for receiving either the audio input signal or the first estimate and arranged for outputting a second estimate of the level of the audio input signal, said second auxiliary level detector having a larger time constant than said first auxiliary level detector,
- computation means for performing a weighting of the first estimate with a weight factor $w$ and the second estimate with a weight factor *1-w,* whereby the weight factor *w* depends on the first and the second estimate, said computation means further arranged for adding the weighted estimations to obtain a resulting estimation of the level of the audio input signal.

Due to the implementation with two auxiliary level detectors with a substantially different time constant, i.e. with a 'fast' and a 'slow' level detector, and the weighting performed on the outputs of the auxiliary level detectors to obtain the resulting estimate of the input signal level, that resulting estimate always falls within the range set by the two auxiliary level detectors. The proposed system indeed provides a level detection that results in behaviour of the compressor that is superior to the behaviour of state-of-the-art compressors by minimizing the amount of distortion that is introduced during the compression, while at the same time providing fast gain changes when needed, for example for protection.

In a preferred embodiment the weight factors are dependent on the difference between the first and the second estimate. The level detection system may then be equipped for computing said difference. When the difference between the auxiliary level detector outputs changes sign, the weight factor is preferably reset to zero.

In an advantageous embodiment the level detection system is arranged for determining the weight by performing a weighting function and for comparing the value returned from the weighting function with a previous value of the weighting factor. The weight factor is then set to the maximum of the weighting function outcome and said previous weighting factor value, while keeping the weighting factor between 0 and 1.

A dynamic range compressor is advantageously provided with a level detection system as previously described.

The invention also relates to a hearing aid, an implantable auditory prosthesis or a mobile telephone device comprising a level detection system as described above.

In another aspect the invention relates to a method for estimating the level of an audio input signal. The method comprises the steps of

- applying the audio input signal to a first auxiliary level detector and obtaining a first estimate of the level of the audio input signal,
- applying either the audio input signal or an output of the first auxiliary level detector to a second auxiliary level detector, said second auxiliary level detector having a larger time constant than the first auxiliary level detector,
- weighting the first estimate with a weight factor *w* and the second estimate with a weight factor *1-w,* said weight factor being dependent on the difference between the first and the second estimate,
- determining an estimation of the level of an audio input signal by summing the weighted first and second estimates.

Preferably the method comprises a step of determining the weight factor *w* comprises the execution of a pre-stored weighting function.

In a further aspect the invention also relates to a method for upgrading an existing level detector system, comprising the steps of

- providing a first auxiliary level detector and a second auxiliary level detector in parallel to the existing level detection system,
- attributing a time constant to the first and the second auxiliary level detector, said first auxiliary level detector having a smaller time constant than said second auxiliary level detector,
- applying a same audio input signal to the existing level detector system and the first and the second auxiliary level detector,
- if the output of the existing level detector system exceeds the output of the first and the second auxiliary level detector, setting said output to the maximum of the outputs of the first and the second auxiliary level detector
- if the output of the existing level detector system stays below the output of the first and the second auxiliary level detector, setting said output to the minimum of the outputs of the first and the second auxiliary level detector.

In a preferred embodiment the first auxiliary level detector is attributed a time constant corresponding to the minimum time constant of the existing level detector system and the second auxiliary level detector is attributed a time constant corresponding to the maximum time constant of the existing level detector system.

The invention also relates to a program, executable on a programmable device containing instructions, which when executed, perform the method as previously described.

For purposes of summarizing the invention and the advantages achieved over the prior art, certain objects and advantages of the invention have been described herein above. Of course, it is to be understood that not necessarily all such objects or advantages may be achieved in accordance with any particular embodiment of the invention. Thus, for example, those skilled in the art will recognize that the invention may be embodied or carried out in a manner that achieves or optimizes one advantage or group of advantages as taught herein without necessarily achieving other objects or advantages as may be taught or suggested herein.

The above and other aspects of the invention will be apparent from and elucidated with reference to the embodiment(s) described hereinafter.

**Brief description of the drawings**

The invention will now be described further, by way of example, with reference to the accompanying drawings, wherein like reference numerals refer to like elements in the various figures.

Fig.1 illustrates a block scheme of an automatic gain control.

Fig.2 illustrates a block diagram of a level detector.

Fig.3 illustrates the output of the level detector of EP1491068 when an artificial signal is applied.

Fig.4 illustrates the output of the level detector of EP1491068 when a speech signal is applied.

Fig.5 illustrates a time constant function as mentioned in EP1491068.

Fig.6 represents an embodiment of the present invention.

Fig.7 illustrates the output of the level detector of the invention when the same artificial signal is applied as in Fig.3.

Fig.8 illustrates the output of the level detector of the invention when the same speech signal is applied as in Fig.4.

Fig.9 illustrates an existing level detector improved with another embodiment of the present invention.

Fig.10 illustrates the output of the level detector of EP1491068, when improved with the present invention, when the same artificial signal as in Fig.3 is applied.

Fig.11 illustrates the output of the level detector of EP1491068, when improved with the present invention, when the same speech signal as in Fig.4 is applied.

**Detailed description of illustrative embodiments**

The present invention will be described with respect to particular embodiments and with reference to certain drawings but the invention is not limited thereto but only by the claims.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment.

Furthermore, while some embodiments described herein include some but not other features included in other embodiments, combinations of features of different embodiments are meant to be within the scope of the invention, and form different embodiments, as would be understood by those in the art.

It should be noted that the use of particular terminology when describing certain features or aspects of the invention should not be taken to imply that the terminology is being redefined herein to be restricted to include any specific characteristics of the features or aspects of the invention with which that terminology is associated.

In the description provided herein, numerous specific details are set forth. However, it is understood that embodiments of the invention may be practiced without these specific details. In other instances, well-known methods, structures and techniques have not been shown in detail in order not to obscure an understanding of this description.

The present invention discloses a level detection system capable of reacting quickly when sudden changes in the input signal require a quick reaction and smoothly when no dramatic changes in the input level are going on and therefore minimize distortion and artefacts.

The invention proposes a solution comprising two auxiliary level detectors, i.e. a fast level detector and a slow level detector. The idea behind the invention is that the output of the level detector system always resides within the range defined by the output of the fast and the slow level detector. Hence, measures are taken to keep the resulting output always between the output levels of the fast and slow level detector separately. The two auxiliary level detectors both have a fixed time constant. The fast level detector uses the input signal as input, while the slow level detector uses either the input signal or the output of the fast level detector as input signal.

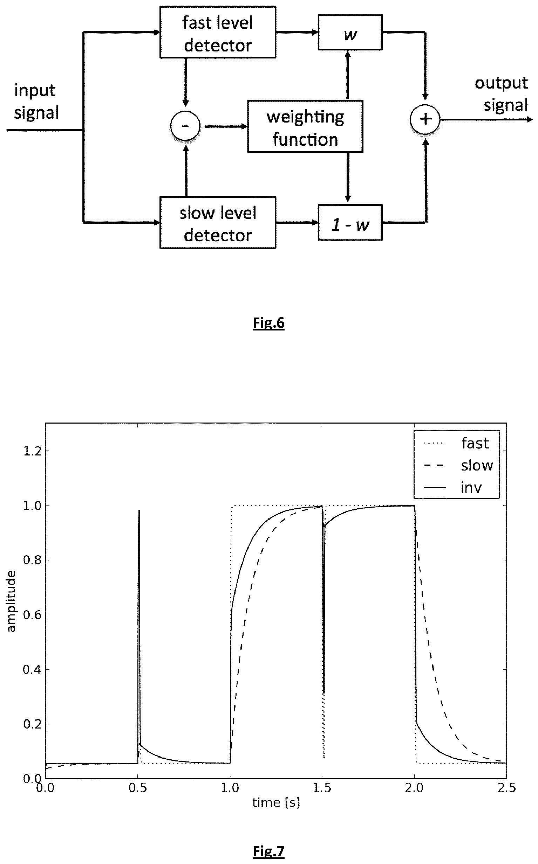

A preferred embodiment of the solution according to the invention is shown in Fig.6. The level detection system according to this invention comprises an auxiliary level detector $L_{fast}$ with a fast time constant and a second auxiliary level detector $L_{slow}$ with a slow time constant. In the embodiment of Fig.6 they are both fed with the input sound signal. In the invention weighted level detection is applied wherein the level at the system's output is determined by a weight factor $w$. The weighted level detector output (i.e. the estimate of the level of the applied input audio signal) is then calculated as a weighted sum of the fast and the slow level detector outputs:

$$L_{weighted} = wL_{fast} + (1-w)L_{slow} \qquad with\ 0 \leq w \leq 1$$

The weighting factor w can be determined in a three-step procedure using its previous value $w_{prev}$ and the two level detectors $L_{fast}$ and $L_{slow}$:

    1. The output of $L_{fast}$ and $L_{slow}$ is determined and the difference between the two level detectors is calculated: $\Delta = L_{fast} - L_{slow}$.

    2. The previous weight $w_{prev}$ is compared to a value derived by the function *weight*($\Delta$) and the current weighting factor value is set to $w$ = max ($w_{prev}$,*weight*($\Delta$)). The function *weight*($\Delta$) is for example given by : *weight*($\Delta$) = 0.06 * *abs*($\Delta$) with a subsequent limitation of *weight*($\Delta$) in the value range of [0,1]. Also non-linear weighting functions can be used.

    3. When the outputs of $L_{slow}$ and $L_{fast}$ cross each other (in other words, $\Delta$ changes its sign), $w$ is reset to zero.

The abrupt change of $w$ in step 3 does not result in an abrupt change of the $L_{weighted}$ output value, because the values of $L_{fast}$ and $L_{slow}$ are similar when $\Delta$ changes its sign.

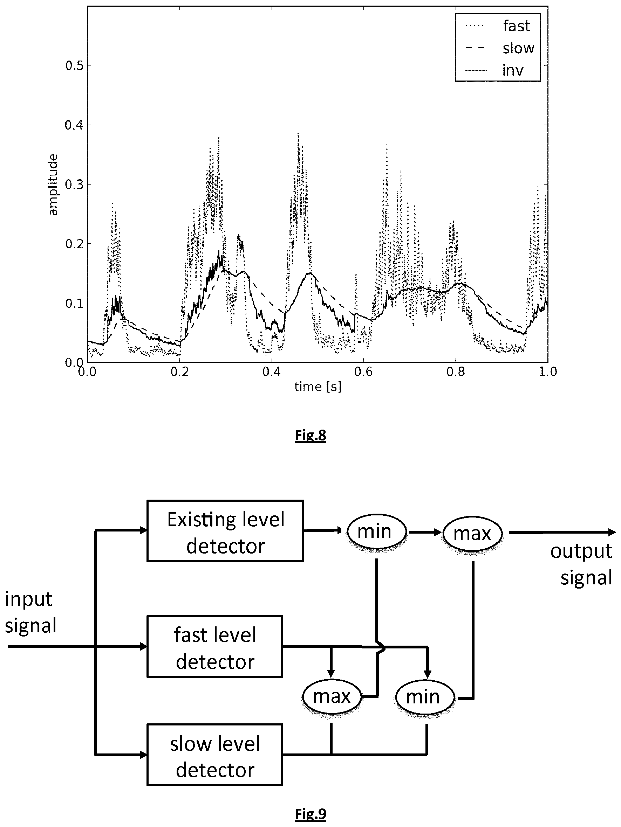

Fig.7 and Fig.8 show the output of the proposed level detection system for the same test signals as used in Fig.3 and Fig.4. It can be observed in both figures that the curve representing the output level determined according to the invention now falls between the curves corresponding to the fast and slow level detectors, respectively.

As mentioned, the idea behind the invention is that the output of the level detector always resides within the range as defined by the output of the fast and the slow level detector. The weight determines if the level detector output is close to the former or the latter, but the restriction of the weight to the range of [0, 1] guarantees that the output of the level detector always resides within the range defined by the output of the fast and the slow level detector.

The invention can be applied to improve any existing level detector. For this purpose, an additional operation is applied at the end of the calculation of the level detector. This procedure forces the output of the level detector to be in the range defined by a fast and a slow level detector. The procedure is illustrated in Fig.9 and can be described in three steps :

    1. Execute in parallel to the level detector a fast level detector that has a small time constant (for example 2 milliseconds) and a slow level detector having a large time constant (for example 200 milliseconds). The input of these level detectors is the same input signal as the input of the level detector to be improved.

    2. If the output of the level detector to be improved is larger than the maximum of the outputs of the fast and the slow level detector, set it to this maximum.

    3. If the output of the level detector to be improved is smaller than the minimum of the outputs of the fast and the slow level detector, set it to this minimum.

Specifically, the invention can be applied to improve the level detector of EP1491068. In this case, the parallel fast level detector can be taken from the auxiliary level detector of EP1491068 and only one additional slow level detector needs to be executed in parallel. Again these two level detectors define a range of allowed values for the output of the level detector of EP1491068. A preferable choice of time constants for these two level detectors is the minimum and maximum of the time constant function used in EP1491068.

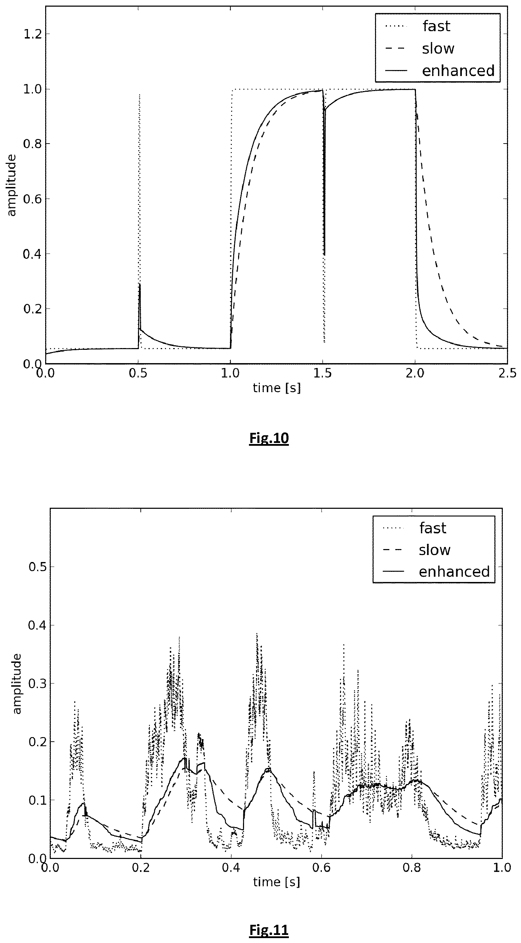

The output of the resulting improved level detector is illustrated in Fig.10 and Fig.11. Although the output of the level detector in Fig.11 seems to be more nervous than in Fig.4, it is closer to the output of the slow level detector. Note also that changes in the output of the level detector are less noticeable because the level changes happen in moments in time in which the input signal is changing. Distortion as a side product of compression is most easily audible if the gain change is applied in a situation of a relatively constant input signal level.

The level detection system of the present invention can be applied in a hearing loss compensation module as described in patent application PCT/EP2011/70606. That hearing loss compensation module comprises an automatic gain control (AGC), which uses a level detector. The level detector system receives at its input a digitized version of the signal picked up by the microphone and the level detector advantageously comprises the level detection system of the present invention. The AGC and the level detector receive their parameters from a control logic block, which forms the 'heart' of the communication device in patent application PCT/EP2011/70606. The control logic block can also receive the parameters of the level detector from a remote server.

While the invention has been illustrated and described in detail in the drawings and foregoing description, such illustration and description are to be considered illustrative or exemplary and not restrictive. The foregoing description details certain embodiments of the invention. It will be appreciated, however, that no matter how detailed the foregoing appears in text, the invention may be practiced in many ways. The invention is not limited to the disclosed embodiments but only by the appended claims.

Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1.   Level detection system for estimating the level of an audio input signal for a dynamic range compressor, comprising

    - a first auxiliary level detector arranged for receiving an audio input signal and for outputting a first estimate of the level of said audio input signal,
    - a second auxiliary level detector arranged for receiving either said audio input signal or said first estimate and arranged for outputting a second estimate of the level of said audio input signal, said second auxiliary level detector having a larger time constant than said first auxiliary level detector, **characterised in that** the level detection system comprises
    - computation means for performing a weighting of said first estimate with a weight factor $w$ and said second estimate with a weight factor $1-w$, said weight factor $w$ being dependent on said first and said second estimate, said computation means further arranged for summing the weighted estimations to obtain a resulting estimation of the level of said audio input signal.

2.   Level detection system as in claim 1, wherein said weight factors are dependent on the difference between said first and said second estimate.

3.   Level detection system as in claim 2, adapted to set said weight factor to zero when said difference changes sign.

4.   Level detection system as in any of claims 1 to 3, further arranged for determining said weight by performing a weighting function and comparing the value returned from said weighting function with a previous value of said weighting factor.

5.   Dynamic range compressor comprising a level detection system as in any of the previous claims.

6.   Hearing aid comprising a level detection system as in any of claims 1 to 4.

7.   Mobile telephone device comprising a level detection system as in any of claims 1 to 4.

8. Implantable auditory prosthesis comprising a level detection system as in any of claims 1 to 4.

9. Method for estimating the level of an audio input signal for a dynamic range compressor, comprising the steps of

- applying said audio input signal to a first auxiliary level detector and obtaining a first estimate of the level of said audio input signal,
- applying either said audio input signal or an output of said first auxiliary level detector to a second auxiliary level detector, said second auxiliary level detector having a larger time constant than said first auxiliary level detector,

**characterised by**

- weighting said first estimate with a weight factor w and said second estimate with a weight factor *1-w,* said weight factor *w* being dependent on the difference between said first and said second estimate,
- determining an estimation of the level of an audio input signal by summing the weighted first and second estimates.

10. Method for estimating the level as in claim 9, comprising a step of determining said weight factor w comprises the execution of a pre-stored weighting function.

11. Method for upgrading an existing level detector system for a dynamic range compressor, comprising the steps of

- providing a first auxiliary level detector and a second auxiliary level detector in parallel to said existing level detection system,
- attributing a time constant to said first and said second auxiliary level detector, said first auxiliary level detector having a smaller time constant than said second auxiliary level detector,
- applying a same audio input signal to said existing level detector system and said first and said second auxiliary level detector,
- if the output of said existing level detector system exceeds the output of said first and said second auxiliary level detector, setting said output to the maximum of the outputs of said first and said second auxiliary level detector,
- if the output of said existing level detector system stays below the output of said first and said second auxiliary level detector, setting said output to the minimum of the outputs of said first and said second auxiliary level detector.

12. Method for upgrading an existing level detector system as in claim 11, wherein said first auxiliary level detector is attributed a time constant corresponding to the minimum time constant of said existing level detector system and said second auxiliary level detector a time constant corresponding to the maximum time constant of said existing level detector system.

13. A program, executable on a programmable device and containing instructions, which when executed, perform the method as in any of the claims 9 to 12.

**Patentansprüche**

1. Pegelerkennungssystem zum Schätzen des Pegels eines Audioeingangssignals für einen Dynamikbereich-Kompressor, umfassend

- einen ersten zusätzlichen Pegeldetektor, der dafür eingerichtet ist, ein Audioeingangssignal zu empfangen, und dafür, eine erste Schätzung des Pegels des Audioeingangssignals auszugeben,
- einen zweiten zusätzlichen Pegeldetektor, der dafür eingerichtet ist, entweder das Audioeingangssignal oder die erste Schätzung zu empfangen, und dafür eingerichtet ist, eine zweite Schätzung des Pegels des Audioeingangssignals auszugeben, wobei der zweite zusätzliche Pegeldetektor eine größere Zeitkonstante aufweist als der erste zusätzliche Pegeldetektor, **dadurch gekennzeichnet, dass** das Pegelerkennungssystem umfasst
- Berechnungsmittel zum Durchführen einer Gewichtung der ersten Schätzung mit einem Gewichtungsfaktor *w* und der zweiten Schätzung mit einem Gewichtungsfaktor *1-w*, wobei der Gewichtungsfaktor *w* von der ersten und der zweiten Schätzung abhängig ist, wobei die Berechnungsmittel weiter dafür eingerichtet sind, die gewichteten Schätzungen zu summieren, um eine resultierende Schätzung des Pegels des Audioeingangssignals zu erhalten.

2. Pegelerkennungssystem nach Anspruch 1, wobei die Gewichtungsfaktoren von der Differenz zwischen der ersten und zweiten Schätzung abhängig sind.

3. Pegelerkennungssystem nach Anspruch 2, das dazu ausgebildet ist, den Gewichtungsfaktor auf Null einzustellen, wenn die Differenz das Vorzeichen ändert.

4. Pegelerkennungssystem nach einem der Ansprüche 1 bis 3, das weiter dafür eingerichtet ist, die Gewichtung über Durchführen einer Gewichtungsfunktion und Vergleichen des von der Gewichtungsfunktion zurückgegebenen Werts mit einem früheren Wert des Gewichtungsfaktors zu bestimmen.

5. Dynamikbereich-Kompressor, welcher ein Pegelerkennungssystem nach einem der vorstehenden Ansprüche umfasst.

6. Hörgerät, welches ein Pegelerkennungssystem nach einem der Ansprüche 1 bis 4 umfasst.

7. Mobiltelefonvorrichtung, welche ein Pegelerkennungssystem nach einem der Ansprüche 1 bis 4 umfasst.

8. Implantierbare Gehörprothese, welche ein Pegelerkennungssystem nach einem der Ansprüche 1 bis 4 umfasst.

9. Verfahren zum Schätzen des Pegels eines Audioeingangssignals für einen Dynamikbereich-Kompressor, umfassend die Schritte des

- Anlegens des Audioeingangssignals an einen ersten zusätzlichen Pegeldetektor, und Erhaltens einer ersten Schätzung des Pegels des Audioeingangssignals,
- Anlegens von entweder dem Audioeingangssignal oder einem Ausgang des ersten zusätzlichen Pegeldetektors an einen zweiten zusätzlichen Pegeldetektor, wobei der zweite zusätzliche Pegeldetektor eine größere Zeitkonstante aufweist als der erste zusätzliche Pegeldetektor,

**gekennzeichnet durch**

- Gewichten der ersten Schätzung mit einem Gewichtungsfaktor $w$ und der zweiten Schätzung mit einem Gewichtungsfaktor $1-w$, wobei der Gewichtungsfaktor $w$ von der Differenz zwischen der ersten und der zweiten Schätzung abhängig ist,
- Bestimmen einer Schätzung des Pegels eines Audioeingangssignals durch Summieren der gewichteten ersten und zweiten Schätzung.

10. Verfahren zum Schätzen des Pegels nach Anspruch 9, umfassend einen Schritt des Bestimmens des Gewichtungsfaktors $w$, welcher das Ausführen einer vorgespeicherten Gewichtungsfunktion umfasst.

11. Verfahren zum Aufrüsten eines bestehenden Pegeldetektorsystems für einen Dynamikbereich-Kompressor, umfassend die Schritte des

- Bereitstellens eines ersten zusätzlichen Pegeldetektors und eines zweiten zusätzlichen Pegeldetektors parallel zum bestehenden Pegelerkennungssystem,
- Zuweisens einer Zeitkonstante zu dem ersten und dem zweiten zusätzlichen Pegeldetektor, wobei der erste zusätzliche Pegeldetektor eine kleinere Zeitkonstante aufweist als der zweite zusätzliche Pegeldetektor,
- Anlegens ein und desselben Audioeingangssignals an das bestehende Pegeldetektorsystem und den ersten und den zweiten zusätzlichen Pegeldetektor,
- Einstellens des Ausgangs auf das Maximum aus den Ausgängen des ersten und des zweiten zusätzlichen Pegeldetektors, wenn der Ausgang des bestehenden Pegeldetektorsystems den Ausgang des ersten und des zweiten zusätzlichen Pegeldetektors übersteigt,
- Einstellens des Ausgangs auf das Minimum aus den Ausgängen des ersten und des zweiten zusätzlichen Pegeldetektors, wenn der Ausgang des bestehenden Pegeldetektorsystems unter dem Ausgang des ersten und des zweiten zusätzlichen Pegeldetektors bleibt.

**12.** Verfahren zum Aufrüsten eines bestehenden Pegeldetektorsystems nach Anspruch 11, wobei dem ersten zusätzlichen Pegeldetektor eine Zeitkonstante zugewiesen wird, die der minimalen Zeitkonstante des bestehenden Pegeldetektorsystems entspricht, und dem zweiten zusätzlichen Pegeldetektor eine Zeitkonstante, die der maximalen Zeitkonstante des bestehenden Pegeldetektorsystems entspricht.

**13.** Programm, welches auf einer programmierbaren Vorrichtung ausführbar ist und Anweisungen enthält, die, wenn sie ausgeführt werden, das Verfahren nach einem der Ansprüche 9 bis 12 durchführen.

## Revendications

**1.** Système de détection de niveau pour estimer le niveau d'un signal d'entrée audio pour un compresseur à plage dynamique, comprenant

- un premier détecteur de niveau auxiliaire agencé pour recevoir un signal d'entrée audio et pour sortir une première estimation du niveau dudit signal d'entrée audio,
- un deuxième détecteur de niveau auxiliaire agencé pour recevoir ledit signal d'entrée audio ou ladite première estimation et agencé pour sortir une deuxième estimation du niveau dudit signal d'entrée audio, ledit deuxième détecteur de niveau auxiliaire ayant une constante de temps plus grande que ledit premier détecteur de niveau auxiliaire, **caractérisé en ce que** le système de détection de niveau comprend
- un moyen de calcul pour effectuer une pondération de ladite première estimation avec un facteur de pondération $w$ et de ladite deuxième estimation avec un facteur de pondération $1-w$, ledit facteur de pondération $w$ dépendant de ladite première et de ladite deuxième estimation, ledit moyen de pondération étant en outre agencé pour additionner les estimations pondérées afin d'obtenir une estimation résultante du niveau dudit signal d'entrée audio.

**2.** Système de détection de niveau selon la revendication 1, dans lequel lesdits facteurs de pondération dépendent de la différence entre ladite première et ladite deuxième estimation.

**3.** Système de détection de niveau selon la revendication 2, adapté pour régler ledit facteur de pondération sur zéro quand ladite différence change de signe.

**4.** Système de détection de niveau selon l'une quelconque des revendications 1 à 3, agencé en outre pour déterminer ladite pondération en effectuant une fonction de pondération et comparer la valeur retournée de ladite fonction de pondération à une valeur précédente dudit facteur de pondération.

**5.** Compresseur à plage dynamique comprenant un système de détection de niveau selon l'une quelconque des revendications précédentes.

**6.** Aide auditive comprenant un système de détection de niveau selon l'une quelconque des revendications 1 à 4.

**7.** Dispositif de téléphone mobile comprenant un système de détection de niveau selon l'une quelconque des revendications 1 à 4.

**8.** Prothèse auditive implantable comprenant un système de détection de niveau selon l'une quelconque des revendications 1 à 4.

**9.** Procédé pour estimer le niveau d'un signal d'entrée audio pour un compresseur à plage dynamique, comprenant les étapes de

- application dudit signal d'entrée audio à un premier détecteur de niveau auxiliaire et obtention d'une première estimation du niveau dudit signal d'entrée audio,
- application dudit signal d'entrée audio ou d'une sortie dudit premier détecteur de niveau auxiliaire à un deuxième détecteur de niveau auxiliaire, ledit deuxième détecteur de niveau auxiliaire ayant une constante de temps plus grande que ledit premier détecteur de niveau auxiliaire,

**caractérisé par**

- la pondération de ladite première estimation avec un facteur de pondération *w* et de ladite deuxième estimation avec un facteur de pondération *1-w*, ledit facteur de pondération *w* dépendant de la différence entre ladite première et de ladite deuxième estimation,
- la détermination d'une estimation du niveau d'un signal d'entrée audio en additionnant les première et deuxième estimations pondérées.

**10.** Procédé pour estimer le niveau selon la revendication 9, comprenant une étape de détermination dudit facteur de pondération *w* comprenant l'exécution d'une fonction de pondération pré-stockée.

**11.** Procédé pour mettre à niveau un système de détection de niveau existant pour un compresseur à plage dynamique, comprenant les étapes de

- fourniture d'un premier détecteur de niveau auxiliaire et d'un deuxième détecteur de niveau auxiliaire parallèlement audit système de détection de niveau existant,
- attribution d'une constante de temps audit premier et audit deuxième détecteur de niveau auxiliaire, ledit premier détecteur de niveau auxiliaire ayant une constante de temps plus petite que ledit deuxième détecteur de niveau auxiliaire,
- application d'un même signal d'entrée audio audit système de détection de niveau existant et audit premier et audit deuxième détecteur de niveau auxiliaire,
- si la sortie dudit système de détection de niveau existant dépasse la sortie dudit premier et dudit deuxième détecteur de niveau auxiliaire, réglage de ladite sortie sur le maximum des sorties dudit premier et dudit deuxième détecteur de niveau auxiliaire,
- si la sortie dudit système de détection de niveau existant reste sous la sortie dudit premier et dudit deuxième détecteur de niveau auxiliaire, réglage de ladite sortie sur le minimum des sorties dudit premier et dudit deuxième détecteur de niveau auxiliaire.

**12.** Procédé pour mettre à niveau un système de détection de niveau existant selon la revendication 11, dans lequel audit premier détecteur de niveau auxiliaire est attribuée une constante de temps correspondant à la constante de temps minimale dudit système de détection de niveau existant et audit deuxième détecteur de niveau auxiliaire une constante de temps correspondant à la constante de temps maximale dudit système de détection de niveau existant.

**13.** Programme, exécutable sur un dispositif programmable et contenant des instructions, qui, lorsqu'elles sont exécutées, effectuent le procédé selon l'une quelconque des revendications 9 à 12.

**Fig.1**

**Fig.2**

**Fig.3**

**Fig.4**

**Fig.5**

**Fig.6**

**Fig.7**

**Fig.8**

**Fig.9**

**Fig.10**

**Fig.11**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1491068 A
- EP 201170606 W